(19) Europäisches Patentamt
European Patent Office
Office européen des brevets

(11) Veröffentlichungsnummer : **0 090 195**
**B1**

(12) # EUROPÄISCHE PATENTSCHRIFT

(45) Veröffentlichungstag der Patentschrift : **21.11.85**

(51) Int. Cl.[4] : **C 07 D451/06, A 61 K 31/46**

(21) Anmeldenummer : **83102109.2**

(22) Anmeldetag : **04.03.83**

(54) **Neue quartäre 6,11-Dihydro-dibenzo-(b,e)-thiepin-11-N-alkyl-norscopinether und Verfahren zu deren Herstellung.**

(30) Priorität : **26.03.82 DE 3211185**

(43) Veröffentlichungstag der Anmeldung : **05.10.83 Patentblatt 83/40**

(45) Bekanntmachung des Hinweises auf die Patenterteilung : **21.11.85 Patentblatt 85/47**

(84) Benannte Vertragsstaaten : **AT BE CH DE FR GB IT LI LU NL SE**

(56) Entgegenhaltungen :
**DE-A- 1 102 167**
**DE-A- 2 414 093**
**Die Akte enthält technische Angaben, die nach dem Eingang der Anmeldung eingereicht wurden und die nicht in dieser Patentschrift enthalten sind.**

(73) Patentinhaber : **BOEHRINGER INGELHEIM KG**

**D-6507 Ingelheim am Rhein (DE)**
**AT BE CH DE FR GB IT LI LU NL SE**
**Boehringer Ingelheim International G.m.b.H**

**D-6507 Ingelheim am Rhein (DE)**
**GB**

(72) Erfinder : **Banholzer, Rolf, Dr.**
**Johann-Calvin-Strasse 11**
**D-6507 Ingelheim/Rhein (DE)**
Erfinder : **Bauer, Rudolf, Dr.**
**Aarstrasse 4**
**D-6200 Wiesbaden (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 090 195 B1**

Jouve, 18, rue St-Denis, 75001 Paris, France

**Beschreibung**

Die Erfindung betrifft neue Verbindungen der allgemeinen Formel I

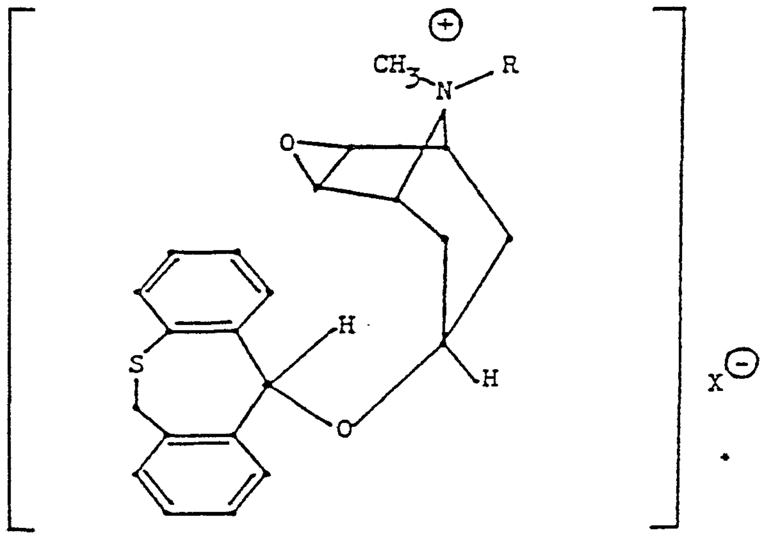

(I)

worin R einen geradkettigen oder verzweigten Alkylrest mit 1-10 Kohlenstoffatomen und $X^{(-)}$ ein pharmakologisch unbedenkliches Anion, wie etwa ein Halogenatom oder den Rest einer organischen Sulfonsäure, darstellt ; Verfahren zu deren Herstellung sowie ihre Verwendung in Pharmazeutika.

Die neuen Verbindungen können auf folgende Weise hergestellt werden :

Umsetzung von Verbindungen der allgemeinen Formel II

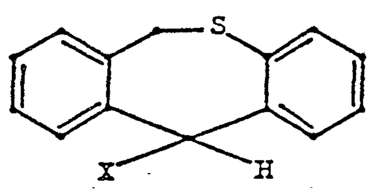

(II)

worin X eine leicht anionisch abspaltbare Gruppe (sog. « leaving group »), wie ein Halogenatom, vorzugsweise ein Chlor- oder Bromatom oder den Rest einer organischen Sulfonsäure, wie den Mesyl- oder Tosylrest, bedeutet, mit Scopin bzw. N-Alkylnorscopinen der allgemeinen Formel III

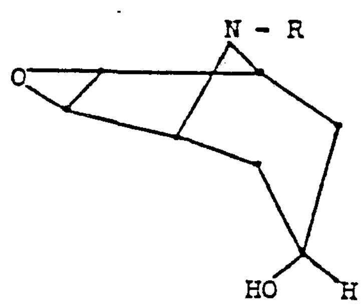

(III)

worin R die oben angegebene Bedeutung hat ; und anschließende Methylierung der so erhaltenen tertiären Verbindungen der allgemeinen Formel Ia

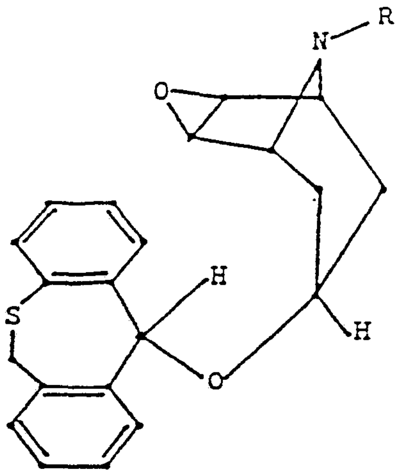

(Ia)

mit üblichen Methylierungsmitteln der allgemeinen Formel IV

$$CH_3—X \qquad (IV)$$

worin X die oben angebenen Bedeutungen hat, behandelt.

Die Ausgangsverbindungen der allgemeinen Formel II sind — ausgehend von Phthalid — über in der Literatur analog beschriebene Verfahren zugänglich, hierzu wird auf die folgenden Publikationen verwiesen :

M. Protiva u. a., Experientia XVIII (7), 326 (1962)
M. Protiva u. a., Collection Czechoslow Chem. Commun. *29*, 2176 (1964)
V. Seidlová u. a., Monatshefte der Chemie *96*, 653 (1965)

Dieser Syntheseweg läuft z. B. nach der nachstehend dargestellten Formelfolge ab :

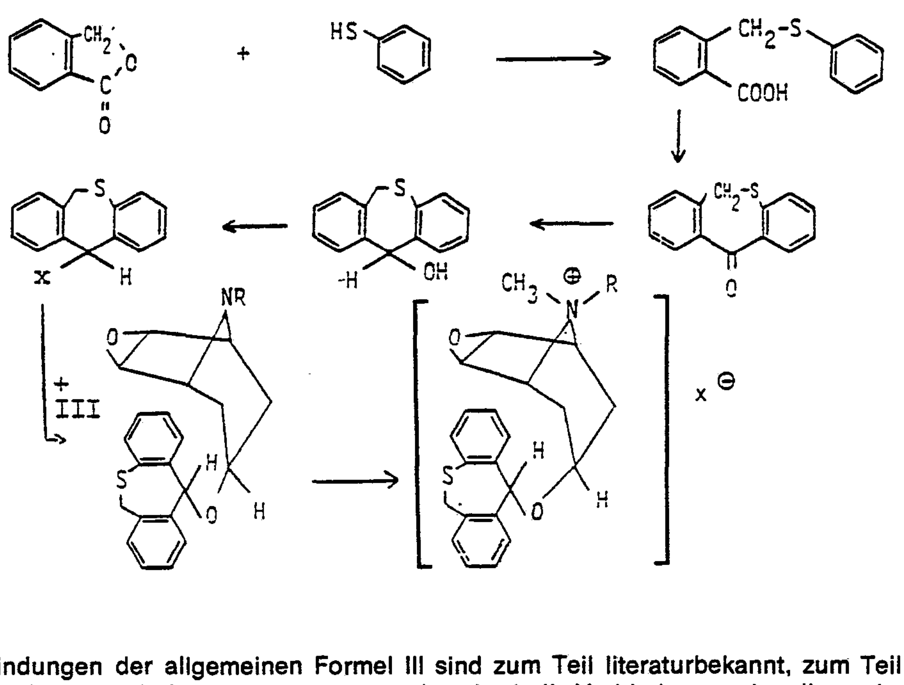

Die Verbindungen der allgemeinen Formel III sind zum Teil literaturbekannt, zum Teil können sie nach an sich bekannten Verfahren gewonnen werden. Auch die Verbindungen der allgemeinen Formel IV sind literaturbekannt.

Die Verbindungen der allgemeinen Formeln I sowie Ia besitzen im Dibenzothiepinrest ein asymmetrisches Kohlenstoffatom und fallen daher bei der oben beschriebenen Synthese als racemisches Gemisch von zwei optisch aktiven Enantiomeren an.

Die neuen quartären Salze haben am isolierten Organ und im Tierversuch am Hund als Aerosol sowohl eine gute anticholinerge als auch eine antihistaminische Wirkung in einem für die Anwendung als Bronchospasmolytika besonders geeigneten, gut ausgewogenen Verhältnis gezeigt und sind dadurch bekannten Broncholytica wie dem Handelsprodukt Ipratropiumbromid überlegen.

Als wirksam haben sich dabei vorzugsweise solche Verbindungen der allgemeinen Formel I erwiesen, bei denen R niederes Alkyl wie Methyl, Ethyl oder Isopropyl bedeutet. Besonders wertvoll ist etwa das 6,11-Dihydrodibenzo-[b,e]-thiepin-scopinether-metho-methan-sulfonat.

Gut wirksam sind auch die entsprechenden tertiären Zwischenprodukte der allgemeinen Formel Ia, jedoch muß hier eher mit Nebenwirkungen wie Mundtrockenheit, Mydriasis etc. gerechnet werden.

Die Messung der Wirksamkeit erfolgte dabei nach folgenden Vorschriften :

a) Spasmolyse am isolierten Organ (Meerschweinchen-Darm) in vitro

Die Versuche werden am isolierten Meerschweinchenrectum nach MAGNUS, R. — Pflügers Arch. *102,* 123 (1904) durchgeführt. — Ein ca. 2 cm langes Stück vom Rectum eines durch Nackenschlag getöteten Meerschweinchens wird in einem Organbad mit Locke-Ringer-Lösung suspendiert. Temperatur des Organbades 35 °C. Die durch das Spasmogen (Acetylcholin bzw. Histamin) ausgelösten Kontraktionen der Längsmuskulatur werden auf einem Kymographen registriert. Das Spasmolytikum bzw. der Vergleichsstandard (z. B. Atropin bzw. Diphenhydramin) werden 90 Sekunden vor der Gabe des Spasmogens dem Organbad zugesetzt (präventive Methode). Sowohl der Vergleichsstandard als auch das zu testende Spasmolytikum werden in steigenden Konzentrationen so dosiert, daß die Wirkung des Spasmogens eine Hemmung zwischen 10 % und 90 % erfährt.

Die Versuche werden an der halbautomatischen Spasmolyseapparatur (Fa. Bundschuh, Griesheim) durchgeführt. Ausgewertet werden nur solche Präparate, für die sich sowohl für den Vergleichsstandard als auch für das zu testende Spasmolytikum am gleichen Darmstück eine Dosiswirkungskurve erstellen läßt.

b) Bronchospasmolyse bei Hunden

Zweck der Untersuchung ist die Prüfung der bronchospasmolytischen Wirkung bei Hunden nach verschiedenen Applikationsformen.

Die Versuche werden an Beagle Hunden beiderlei Geschlechts mit einem Körpergewicht von 10-14 kg durchgeführt. Als Narkotikum dient ein Gemisch von Chloralose-Urethan (8 g Chloralose, 40 g Urethan in 100 ml Aqua dest.), das in einer Dosis von 1,5 ml/kg i.v. injiziert wird.

Operationstechnik

Grundvoraussetzung für die von KONZETT, H. und R. RÖSSLER Arch. exper. Path. und Pharmakol. *195,* 71 (1940) beschriebene Versuchsanordnung ist ein absolut dichtes Registriersystem. Die Versuchshunde werden zunächst mit einem mit aufblasbarer Manschette versehenen Trachealtubus intubiert. Der Blutdruck wird blutig in der linken A. carotis mittels eines Statham-Transducers gemessen. Zur Erleichterung der i.v. Applikation bindet man einen Katheter mit einem definierten Fassungsvermögen in die rechte V. femoralis ein. Vor Eröffnung des Thorax im Bereich des Proc. xiphoideus wird das Versuchstier an die Atempumpe (Fa. Havard, Modell 607) angeschlossen. Die Atemfrequenz beträgt 16-18/min, der Beatmungsdruck 12-16 cm $H_2O$. — Wie bei der Original-Methode nach KONZETT und RÖSSLER werden beide Vagi durchtrennt ; der Bronchospasmus wird durch rasche i.v. Injektion von Acetylcholin bzw. Histamin in Intervallen von 5 Minuten erzeugt. Die Registrierung von Blutdruck, Bronchospasmus und Herzfrequenz erfolgt auf einem Grass Polygraphen.

a) Nach i.v. Gabe

Nach Erreichen konstanter Spasmen kann die zu prüfende Substanz bzw. der Vergleichsstandard i.v. gegeben werden. Die Substanzgabe erfolgt stets 1 Minute vor der Acetylcholin- bzw. Histamininjektion.— Ausgewertet wird die prozentuale Hemmung der Spasmen und die Wirkungsdauer (Halbwertszeit), d. h. Zeit, die verstreicht, bis nur noch die Hälfte der Substanzwirkung vorhanden ist.

b) Nach Gabe von wäßrigem Aerosol

Um Aerosole bei der beschriebenden Versuchsanordnung anwenden zu können, wird zwischen Atempumpe und Versuchstier, möglichst nahe der Trachealkanüle eine Woulf' sche Flasche mit 5 Liter Fassungsvermögen geschaltet. Diese ist mit Dreiwegehähnen so versehen, daß für die Beatmung folgende Möglichkeiten bestehen :

a. Atempumpe — 1. Dreiwegehahn — Zwischenstück — 2. Dreiwegehahn — Versuchstier.

b. Atempumpe — 1. Dreiwegehahn — Woulf' sche Flasche — 2. Dreiwegehahn — Versuchstier.

Der direkte Weg (a) dient der Beatmung während des Versuches, wogegen der Weg über die Flasche (b) bei der Zuführung des Aerosols benutzt wird.

Herstellung und Applikation des Aerosols

Der Auslaßstutzen einer Inhalette (Fa. Dräger, Lübeck, Modell M 12123) wird durch einen

Gummistopfen geführt, der in die untere Schliffföffnung der Woulf' schen Flasche paßt. Nun wird die wäßrige Lösung der zu testenden Substanz vernebelt und das Aerosol in die Flasche geblasen. Vernebelt wird 30 sec mit einem $O_2$-Fluß von 15 l/min. Um bei der Applikation eine Durchmischung von Raumluft und Aerosol in der Flasche zu vermeiden, wird der Einlaßteil der Woulf'schen Flasche mit einem Kondomgummi verbunden. Die von der Atempumpe gelieferte Luft bläht den Kondomgummi auf und verdrängt aus der Flasche das Aerosol, das dann dem Versuchstier zugeführt wird. Nach der Applikation werden die Dreiwegehähne wieder so umgelegt, daß der Beatmungsweg an der Flasche vorbeiführt. — Bei jeder Applikation werden 1,5 Minuten vor der Acetylcholininjektion 5 Atemhübe Aerosol gegeben.

Ausgewertet wird die prozentuale Hemmung des Acetylcholinspasmus, der Eintritt des Wirkungsmaximums, die Halbwertszeit sowie die Wirkung auf den durch die Acetylcholingabe bedingten Blutdruckabfall.

Statistische Auswertung

Mit der Methode der kleinsten Quadrate wurde den Werten eine lineare und quadratische Funktion angepaßt. Die Beiträge der einzelnen Ansätze wurden varianzanalytisch auf Signifikanz geprüft. Nach Finney erfolgte die Berechnung der $ED_{50}$ — bzw. der $EC_{50}$ — Werte mit dem zugehörigen Vertrauensbereich ($P = 0,95$).

Die erfindungsgemäßen Verbindungen der allgemeinen Formel I sind aufgrund ihrer pharmakologischen Eigenschaften gut geeignet für die Behandlung von Spasmen und Bronchialspasmen. Als Einzeldosis für die orale Anwendung kommt dabei eine Wirkstoffmenge von 5-350 mg in Betracht.

Geeignete pharmazeutische Anwendungsformen sind beispielsweise Tabletten, Kapseln, Zäpfchen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, oder Magnesiumstearat oder Talk, und/oder Mitteln zur Erzielung eines Depoteffekts, wie Carboxypolymethylen, Carboxymethylcellulose, Celluloseacetatphthalat oder Polyvinylacetat, erhalten werden.

Die Tabletten können auch aus mehreren Schichten bestehen.

Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffekts aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmackverbesserndes Mittel, z. B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie 1-Hydroxybenzoate, enthalten.

Die einen oder mehrere Wirkstoffe bzw. Wirkstoffkombinationen enthaltenden Kapseln können beispielsweise hergestellt werden, indem man die Wirkstoffe mit inerten Trägerm, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol bzw. dessen Derivaten, herstellen.

Zur Behandlung bronchospasmolytischer Atemwegserkrankungen werden die Wirkstoffe in üblicher Weise zu Aerosolen verarbeitet und in Spraydosen (vorzugsweise mit Dosiereinrichtung) abgefüllt. Als therapeutische Einzeldosis kommt hier eine Menge von 5-500 $\gamma$, vorzugsweise 50-250 $\gamma$ in Frage, entsprechend einem Wirkstoffgehalt von 0,007-1 %.

Die erfindungsgemäßen Wirkstoffe können auch mit anderen Wirkstofen, z. B. Mucolytica wie Bromhexin oder Cystein, gemischt und in Form von sog. Kombinationspräparaten verabreicht werden.

Die folgenden Beispiele erläutern die Erfindung, ohne sie zu beschränken :

A. Herstellungsbeispiele :

Beispiel 1

a) 6,11-Dihydro-dibenzo-[b, e]-thiepin-11-scopinether

195,5 g (1,26 Mol) Scopin
[Herstellung möglich in Analogie und nach G. Werner
u. a. Tetrahedron Letters *14,* 1283 (1967), R. Willstätter
u. a. Ber. dtsch. chem. Ges. *56,* 1079 (1923), J. Meinwald
u. a. J. Amer. chem. Soc. *79,* 665, (1957), H. L. Schmidt
u. a. Liebigs Ann. Chem. *688,* 228 (1965)]

werden in 600 ml abs. Methylenchlorid gelöst und zu dieser Lösung bei 40 °C eine Lösung von 156,5 g (0,63 Mol) 11-Chlor-6,11-dihydro-dibenzo-[b,e]-thiepin (Herstellung gemäß den obenbezeichneten Publikationen von M. Protiva u. a., bzw. V. Seidlová u. a.).
in 200 ml abs. Methylenchlorid innerhalb drei Stunden getropft. Nach drei Tagen bei 40 °C wird die Umsetzung abgebrochen, die ausgefallenen Kristalle (Scopin-hydrochlorid 110 g) werden abgesaugt und mit Methylenchlorid gewaschen. Die vereinigten Methylenchloridlösungen werden darauf mit Wasser, dann mit 600 ml 1 N Salzsäure extrahiert, die Methylenchloridphase mit Wasser neutral gewaschen und die gesammelten Wasserphasen mit einer wäßrigen Natriumcarbonatlösung (66 g Natriumcarbonat/200 ml Wasser) alkalisch gestellt und mit Methylenchlorid extrahiert. Die gesammelten alkalischen Methylenchloridphasen werden über Natriumsulfat getrocknet und das Methylenchlorid unter vermindertem Druck abdestilliert.

Der Destillationsrückstand : 189 g weiße Kristalle, wird aus Acetonitril (über Aktivkohle) umkristallisiert.

Ausbeute : 159,8 g (69,4 % d. Th.).

Weiße Kristalle Schmelzpunkt. 174-175 °C.

Das Vorliegen dieser Verbindung wird durch Elementaranalyse und Spektren bestätigt.

b) 6,11-Dihydro-dibenzo-[b, e]-thiepin-11-scopinethermethobromid

2,0 g (0,005 5 Mol) des wie vorstehend beschrieben erhaltenen 6,11-Dihydro-dibenzo-[b, e]-thiepin-11-scopinethers werden in 35 ml absolutem Acetonitril bei Raumtemperatur mit 2,6 g (0,027 5 Mol) Methylbromid umgesetzt. Nach 24 Stunden wird die Quaternierung abgebrochen. Es hat sich gezeigt, daß bei der Herstellung der Methobromide in größerem Maßstab relativ lange Reaktionszeiten und ein größerer Überschuß an Methylbromid zweckmäßig ist.

Das Acetonitril wird abdestilliert, der Rückstand in Acetonitril aufgenommen und mit Ether übersättigt. Nach mehrmaliger Wiederholung dieses Verfahrens wird das feste Produkt in Wasser gelöst, mit Aktivkohle gereinigt und die klare Lösung anschließend gefriergetrocknet.

Ausbeute : 1,2 g (47,4 % d. Th.).

Weiße Substanz vom Schmelzpunkt ab 140 °C (unter Erweichen).

Laut Elementaranalyse und Spektren liegt diese Verbindung als Monohydrat vor.

## Beispiel 2

6,11-Dihydro-dibenzo-[b, e]-thiepin-11-scopinethermethomethansulfonat

131,3 g (0,36 Mol) des nach Beispiel 1 a) hergestellten 6,11-Dihydro-dibenzo-[b, e]-thiepin-11-scopinethers werden in 400 ml abs. Methylenchlorid gelöst, dazu 47,4 g (0,43 Mol) Methansulfonsäuremethylester gegeben und bei 40 °C unter Rühren umgesetzt. Nach 6 Stunden werden weitere 19 g (0,17 Mol) Methansulfonsäuremethylester hinzugegeben. Nach einer Gesamtreaktionsdauer von 24 Stunden wird die Quaternierung abgebrochen. Die ausgefallenen Kristalle werden abgesaugt und mit Methylenchlorid gewaschen. 1. Rohkristalle 149,6 g, weiße Kristalle, Schmelzpunkt 236-237 °C (Zersetzung). Zur Methylenchloridlösung werden weitere 8,5 g (0,08 Mol) Methansulfonsäuremethylester gegeben und nochmals 24 Stunden lang bei 40 °C umgesetzt. Eine vollständige Umsetzung macht jedoch eine nochmalige Zugabe von 8,5 g (0,08 Mol) Methansulfonsäuremethylester und eine Reaktionsdauer von weiteren 24 Stunden erforderlich. Nach dem Waschen mit Methylenchlorid werden 2. Rohkristalle von 18,1 g, weiße Kristalle erhalten, Schmelzpunkt 236-237 °C (Zersetzung). Beide Rohkristallfraktionen werden zusammen in Methanol über Aktivkohle gereinigt und die methanolische Lösung nach Zugabe von Acetonitril unter vermindertem Druck konzentriert bis Kristallisation eintritt.

Ausbeute : 137,7 g (80,4 % d. Th.).

Weiße Kristalle, Schmelzpunkt 236-237 °C (Zersetzung).

Das Vorliegen dieser Verbindung wird durch Elementaranalyse und Spektren bestätigt.

## Beispiel 3

a) 6,11-Dihydro-dibenzo-[b, e]-thiepin-N-ethylnorscopinether

Bei der Herstellung dieser Verbindung kann wie im Beispiel 1a) verfahren werden. N-Ethylnorscopin erhält man in Analogie zu den für Scopin in Beispiel 1a) angegebenen Verfahren aus dem entsprechenden N-Ethylnorscopolamin.

5,5 g (0,032 Mol) N-Ethylnorscopin werden in 50 ml absolutem Toluol gelöst und zu dieser Lösung bei 80 °C eine Lösung von 4,0 g (0,016 Mol) 11-Chlor-6,11-dihydro-dibenzo-[b, e]-thiepin in 30 ml absolutem Toluol innerhalb 10 Minuten getropft. Nach 6 Stunden bei 80 °C wird die Umsetzung abgebrochen. Bei der Aufarbeitung wird wie im Beispiel 1 verfahren.

Ausbeute : 5,7 g (93,4 % d. Th.).

Weiße Kristalle (Acetonitril), Schmelzpunkt 128-130 °C.

Das Vorliegen dieser Verbindung wird durch Elementaranalyse und Spektren bestätigt.

b) 6,11-Dihydro-dibenzo-[b, e]-thiepin-11-N-ethylnorscopinether-methobromid

2,0 g (0,005 3 Mol) des in der vorhergehenden Stufe hergestellten 6,11-Dihydro-dibenzo-[b, e]-thiepin-11-N-ethylnorscopinethers werden in 14 ml absolutem Acetonitril bei Raumtemperatur mit 3,75 g (0,04 Mol) Methylbromid umgesetzt. Nach 48 Stunden wird die Quaternierung abgebrochen. Das Acetonitril wird abdestilliert, der Rückstand in Acetonitril aufgenommen und mit Ether übersättigt. Nach mehrmaliger Wiederholung dieses Verfahrens wird das feste Produkt in Wasser gelöst, mit Aktivkohle gereinigt und die klare Lösung anschließend gefriergetrocknet.

Ausbeute : 1,5 g (60,0 % d. Th.).

Weiße Festsubstanz, Schmelzpunkt ab 130 °C, unter Erweichen.

Laut Elementaranalyse und Spektren liegt diese Verbindung als Halbhydrat vor.

Weiter lassen sich nach den vorstehenden Vorschriften herstellen :

Beispiel 4

6,11-Dihydro-dibenzo-[b, e]-thiepin-11-N-ethylnorscopinether-methomethansulfonat (analog Beispiel 2)

Beispiel 5

6,11-Dihydro-dibenzo-[b, e]-thiepin-N-n-propylnorscopinether und das-methobromid bzw. -methomethansulfonat (analog den Beispielen 1 bzw. 2)

Beispiel 6

6,11-Dihydro-dibenzo-[b, e]-thiepin-N-isopropylnorscopinether und das-methobromid bzw. -methomethansulfonat (analog den Beispielen 1 und 2)

B. Pharmazeutische Zubereitungen :

A) Tabletten

1 Tablette enthält :

| | |
|---|---|
| 6,11-Dihydro-dibenzo-[b, e]-thiepin-N-ethylnorscopinether-methobromid | 10,0 mg |
| Milchzucker | 76,0 mg |
| Kartoffelstärke | 30,0 mg |
| Gelatine | 3,0 mg |
| Magnesiumstearat | 1,0 mg |
| | 120,0 mg |

Herstellungsverfahren :

Die Wirksubstanz wird mit der zehnfachen Menge Milchzucker intensiv verrieben. Man mischt diese Verreibung mit dem restlichen Milchzucker sowie mit Kartoffelstärke und granuliert mit einer 10 %igen wäßrigen Lösung Gelatine durch Sieb 1,5 mm. Trocknung bei 40 °C. Das getrocknete Granulat wird nochmals durch Sieb 1 mm gerieben und mit Magnesiumstearat vermischt. Aus der Mischung werden Tabletten gepreßt.

Tablettengewicht : 120 mg

Stempel : 7 mm flach mit Teilkerbe.

B) Dragées

1 Dragéekern enthält :

| | |
|---|---|
| 6,11-Dihydro-dibenzo [b, e]-thiepin-11-sopinether-methomethansulfonat | 10,0 mg |
| Milchzucker | 23,0 mg |
| Maisstärke | 14,5 mg |
| Polyvinylpyrrolidon | 2,0 mg |
| Magnesiumstearat | 0,5 mg |
| | 50,0 mg |

Herstellungsverfahren :

Die Wirksubstanz wird mit der zehnfachen Menge Milchzucker intensiv verrieben, mit dem restlichen

Milchzucker sowie mit der Maisstärke gemischt und mit einer 15 %igen wäßrigen Lösung des Polyvinylpyrrolidons durch Sieb 1 mm granuliert. Die bei 40 °C getrocknete Masse wird nochmals durch obiges Sieb gerieben, mit Magnesiumstearat gemischt und anschließend zu Dragéekernen verpreßt.

Kerngewicht : 50 mg

Stempel : 5 mm gewölbt.

Die so hergestellten Dragéekerne werden nach bekannten Verfahren mit einer Hülle überzogen, die im wesentlichen aus Zucker und Talkum besteht. Die fertigen Dragées werden mit Hilfe von Bienenwachs poliert. Dragéegewicht : 85 mg.

C) Tropfen

Zusammensetzung :

| | |
|---|---|
| 6,11-Dihydro-dibenzo-[b, e]-thiepin-11-scopinether-methobromid | 0,012 5 g |
| Saccharin-Natrium | 0,3 g |
| Sorbinsäure | 0,1 g |
| Äthanol | 30,0 g |
| Aromastoffe | 1,0 g |
| Dest. Wasser | ad 100,0 g |

Herstellungsverfahren :

Man mischt die Lösung der Wirksubstanz und der Likõressenz in Äthanol mit der Lösung der Sorbinsäure und Saccharin in Wasser und filtriert faserfrei.

1 ml Tropfenlösung enthält 0,125 mg.

D) Ampullen

1 Ampulle enthält :

| | |
|---|---|
| 6,11-Dihydro-dibenzo-[b, e]-thiepin-11-N-ethylnorscopinether-methomethansulfonat | 0,5 mg |
| Weinsäure | 150,0 mg |
| Dest. Wasser | ad 3,0 ml |

Herstellungsverfahren :

In destilliertem Wasser werden nacheinander Weinsäure, Polyäthylenglykol und die Wirksubstanz gelöst. Man füllt mit destilliertem Wasser auf das gegebene Volumen auf und filtriert keimfrei.

Abfüllung : in weiße 3 ml-Ampullen unter Stickstoffbegasung

Sterilisation : 20 Minuten bei 120 °C.

E) Suppositorien

1 Zäpfchen enthält :

| | |
|---|---|
| 6,11-Dihydro-dibenzo-[b, e]-thiepin-N-isopropylnorscopinether-methobromid | 0,25 mg |
| Milchzucker | 4,75 mg |
| Zäpfchenmasse (z. B. Witepsol W 45) | 1 695,0 mg |

Herstellungsverfahren :

Die Verreibung der Wirksubstanz mit Milchzucker wird mit Hilfe eines Eintauchhomogenisators in die geschmolzene und auf 40 °C abgekühlte Zäpfchenmasse eingerührt. Man kühlt auf 37 °C ab und gießt in leicht vorgekühlte Formen.

Zäpfchengewicht : 1,7 g.

F) Dosieraerosol

| | |
|---|---|
| 6,11-Dihydro-dibenzo-[b, e]-thiepin-11-scopinether-methobromid | 0,007-0,7 % |
| Oberflächenaktiver Stoff z. B. Sorbitantrioleat | 0,5 -2,0 % |
| Monofluortrichlor- und Difluordichlormethan 40 : 60 | ad 100 % |
| Einzeldosierung je Betätigung | 20-150 γ |

**Patentansprüche**

1. Verbindungen der allgemeinen Formel I

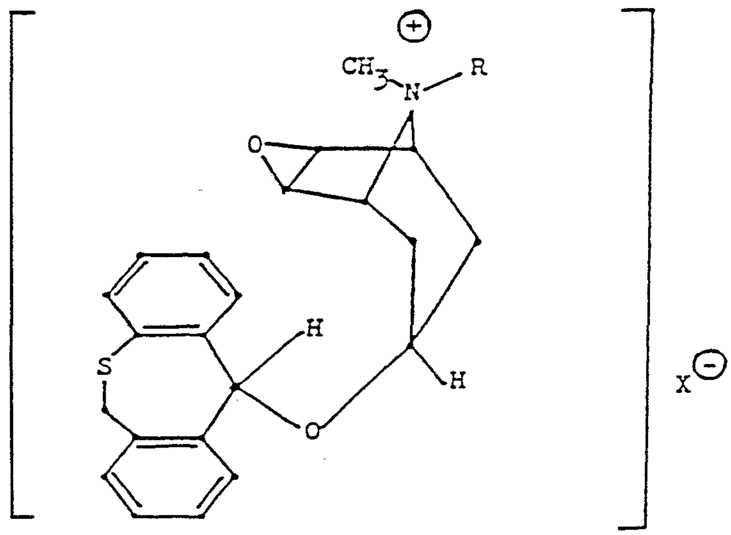

(I)

worin R einen geradkettigen oder verzweigten Alkylrest mit 1-10 Kohlenstoffatomen und $X^{\ominus}$ ein pharmakologisch unbedenkliches Anion darstellt.

2. Verbindungen der allgemeinen Formel I, worin R einen geradkettigen oder verzweigten Alkylrest mit 1-3 C-Atomen darstellt und $X^{\ominus}$ die obengenannte Bedeutung hat.

3. 6,11-Dihydro-dibenzo-[b, e]-thiepin-11-scopinethermetho-methansulfat.

4. Verbindungen der allgemeinen Formel Ia

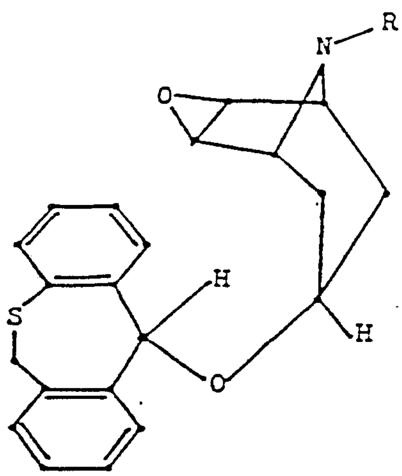

(Ia)

in der R einen geradkettigen oder verzweigten Alkylrest mit 1-10 Kohlenstoffatomen darstellt, sowie deren Säureadditionssalze mit einem pharmakologisch unbedenklichen Anion.

5. Verfahren zur Herstellung von Verbindungen der allgemeinen Formel I

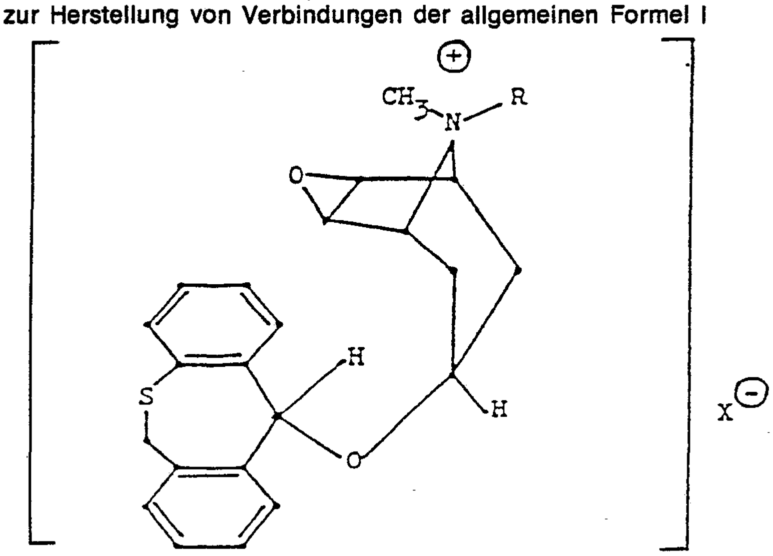

(I)

dadurch gekennzeichnet, daß man Verbindungen der allgemeinen Formel II

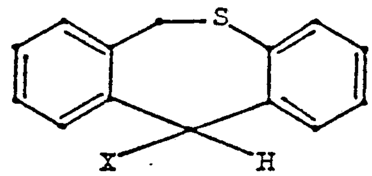

(II)

worin X eine leicht abspaltbare Gruppe bedeutet, mit Verbindungen der allgemeinen Formel III

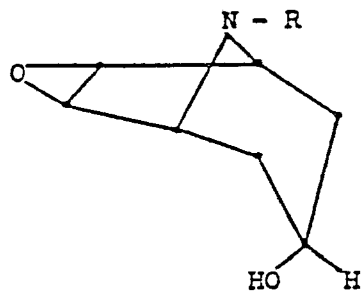

(III)

worin R die oben angegebene Bedeutung hat, umsetzt und anschließend die so erhaltenen Verbindungen der allgemeinen Formel Ia

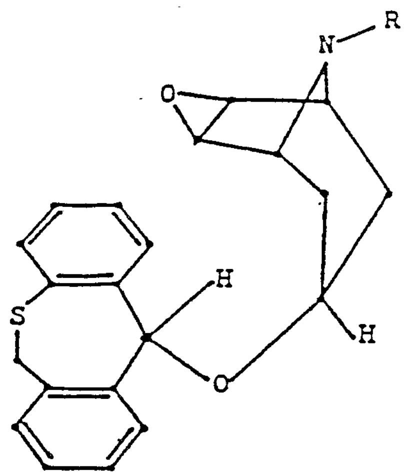

(Ia)

mit üblichen Methylierungsmitteln der allgemeinen Formel IV

$CH_3$—X (IV)

worin X die oben angegebenen Bedeutungen hat, behandelt.

6. Verfahren zur Herstellung von Verbindungen der allgemeinen Formel Ia

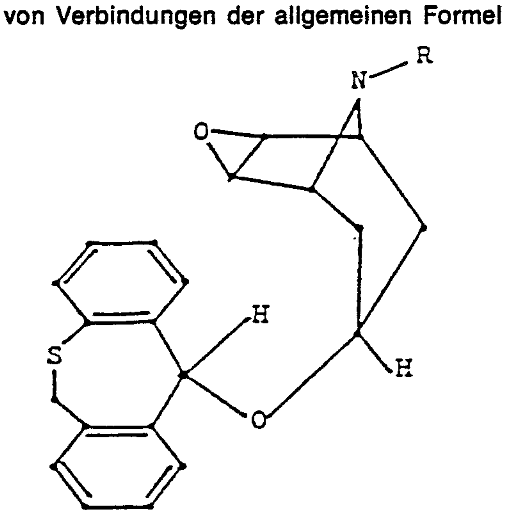

(Ia)

dadurch gekennzeichnet, daß man Verbindungen der allgemeinen Formel II

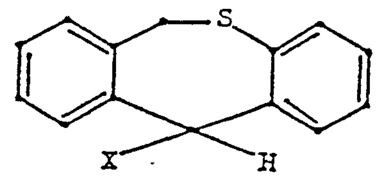

(II)

worin X eine leicht abspaltbare Gruppe bedeutet, mit Verbindungen der allgemeinen Formel III

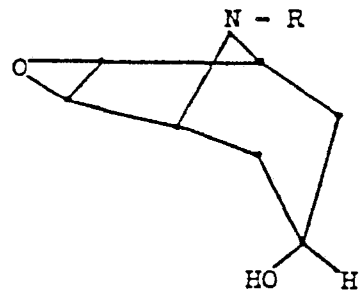

(III)

worin R die oben angegebene Bedeutung hat, umsetzt.

7. Pharmazeutische Präparate, gekennzeichnet durch einen Gehalt an Verbindung(en) der allgemeinen Formel I.

8. Verfahren zur Herstellung pharmazeutischer Präparate nach Anspruch 7, dadurch gekennzeichnet, daß man einen oder mehrere Verbindungen der allgemeinen Formel I mit üblichen galenischen Hilfs- und/oder Trägerstoffen in üblicher Weise vermischt und zu Arzneimitteln verarbeitet.

9. Verwendung von Verbindungen der allgemeinen Formel I zur Herstellung broncholytischer Arzneimittel.

**Claims**

1. Compounds of general formula I

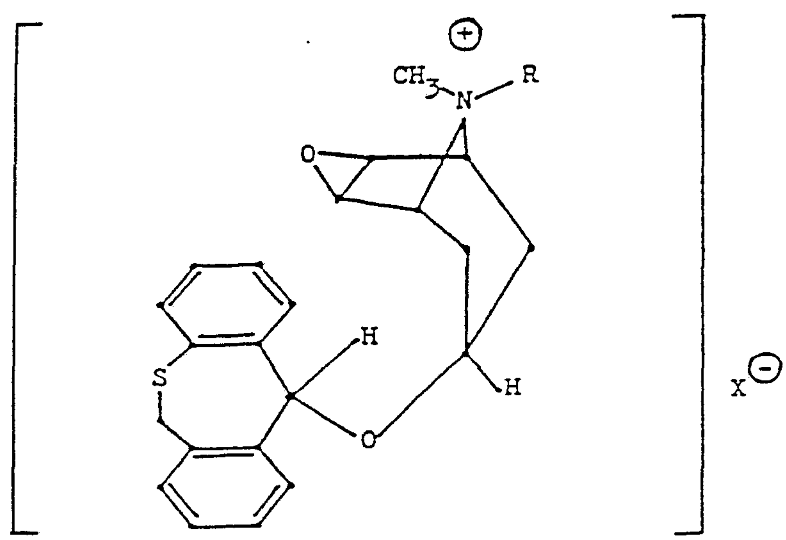

wherein R represents a straight-chained or branched alkyl group with 1 to 10 carbon atoms and $X^{\ominus}$ represents a pharmacologically harmless anion.

2. Compounds of general formula I wherein R represents a straight-chained or branched alkyl group with 1-3 carbon atoms and $X^{\ominus}$ is as hereinbefore defined.

3. 6,11-Dihydro-dibenzo-[b, e]-thiepine-11-scopine ether-metho-methanesulphate.

4. Compounds of general formula Ia

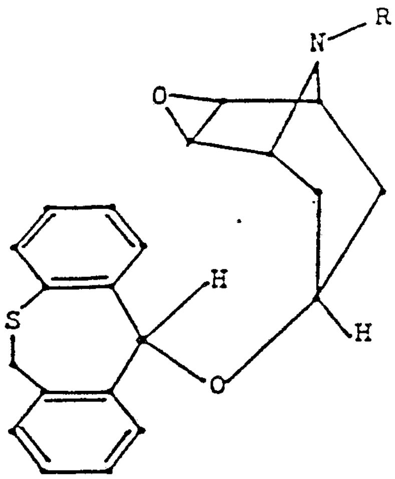

(Ia)

wherein R represents a straight-chained or branched alkyl group with 1 to 10 carbon atoms, and the acid addition salts thereof with a pharmacologically harmless anion.

5. Process for preparing compounds of general formula I

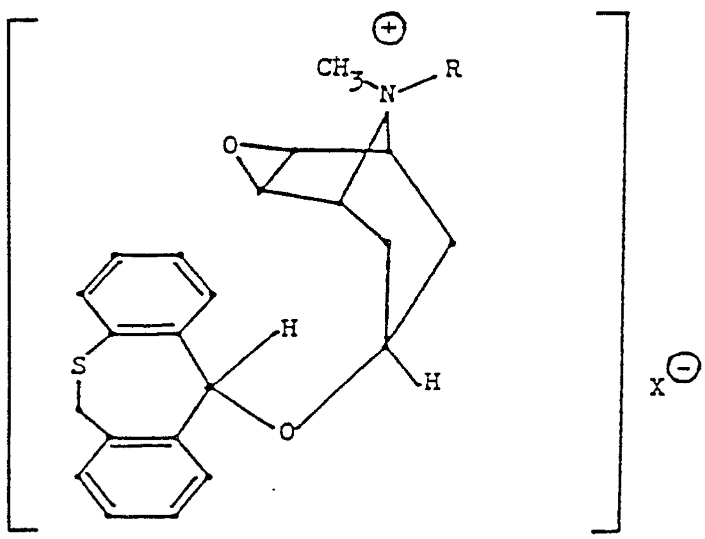

(I)

characterised in that compounds of general formula II

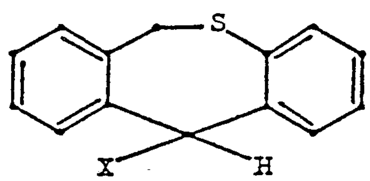

(II)

wherein X represents an easily removable group are reacted with compounds of general formula III

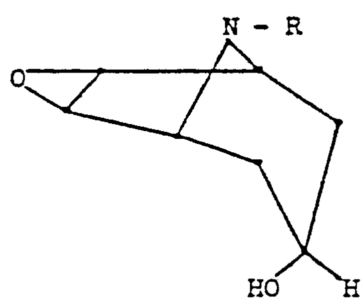

(III)

wherein R is as hereinbefore defined, and subsequently the compounds of general formula Ia

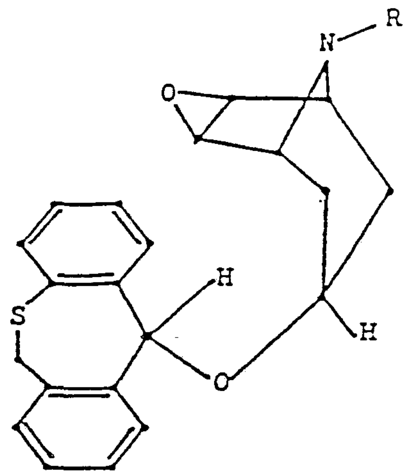

(Ia)

thus obtained are treated with conventional methylating agents of general formula IV

$$CH_3—X \quad (IV)$$

wherein X is as hereinbefore defined.

6. Process for preparing compounds of general formula Ia

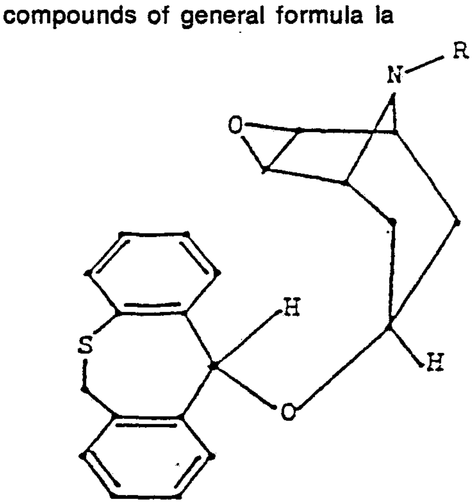

(Ia)

characterised in that compounds of general formula II

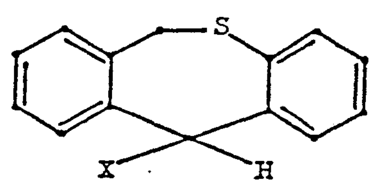

(II)

wherein X represents an easily removable group are reacted with compounds of general formula III

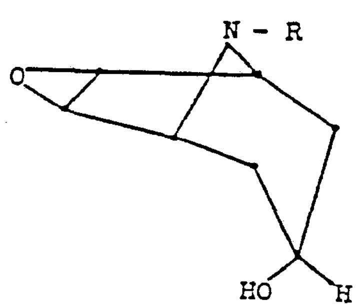

(III)

wherein R is as hereinbefore defined.

7. Pharmaceutical preparations, characterised in that they contain a compound or compounds of general formula I.

8. Process for preparing pharmaceutical preparations as claimed in claim 7, characterised in that one or more compounds of general formula I are mixed with conventional galenic excipients and/or carriers in the usual way and processed to form pharmaceutical compositions.

9. Use of compounds of general formula I for preparing broncholytic pharmaceutical compositions.

**Revendications**

1. Composés de formule générale I

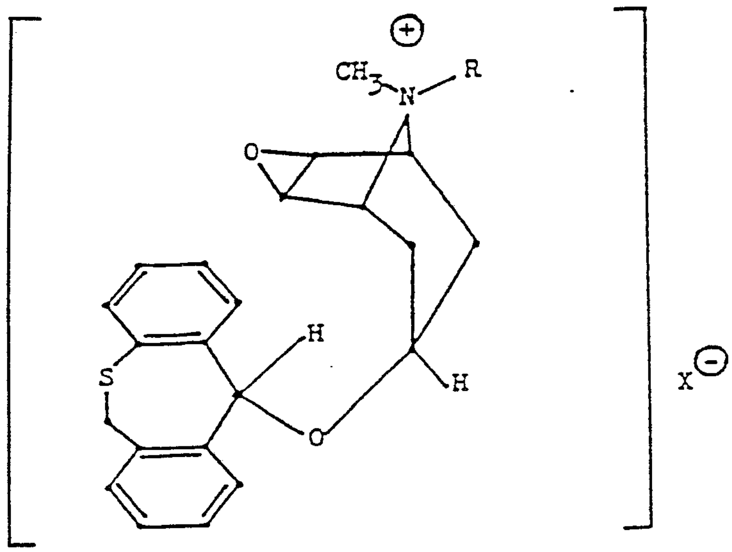

(I)

dans laquelle R représente un radical alcoyle rectiligne ou ramifié avec 1-10 atomes de carbone et $X^{\ominus}$ un anion pharmacologiquement inoffensif.

2. Composés de formule générale I dans laquelle R représente un radical alcoyle rectiligne ou ramifié avec 1-3 atomes de C et $X^{\ominus}$ a la signification mentionnée plus haut.

3. Le méthométhanesulfonate de 6,11-dihydrodibenzo-[b, e]-thiépine-11-scopinéther.

4. Composés de formule générale Ia

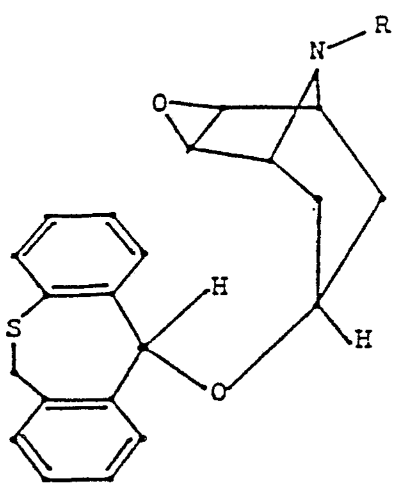

(Ia)

dans laquelle R représente un radical alcoyle rectiligne ou ramifié avec 1-10 atomes de carbone, ainsi que ses sels d'addition d'acides avec un anion pharmacologiquement inoffensif.

5. Procédé pour la préparation de composés de formule générale I

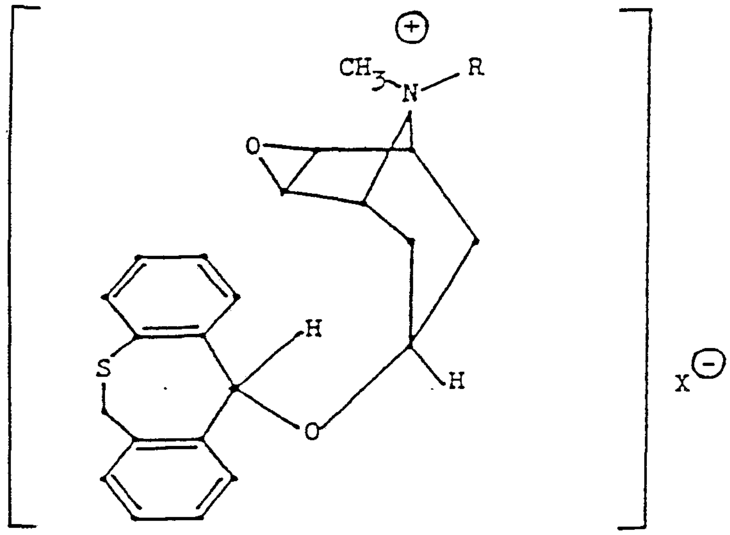

(I)

caractérisé en ce qu'on fait réagir des composés de formule générale II

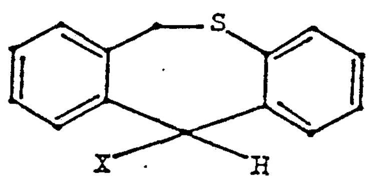

(II)

dans laquelle X représente un groupe facilement clivable, avec des composés de formule générale III

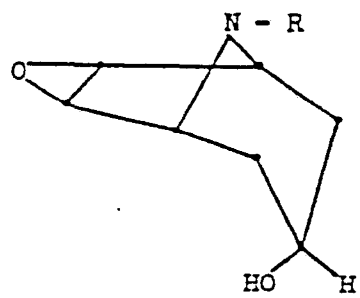

(III)

dans laquelle R a la signification indiquée plus haut et en ce qu'ensuite, on traite les composés ainsi obtenus de formule générale Ia

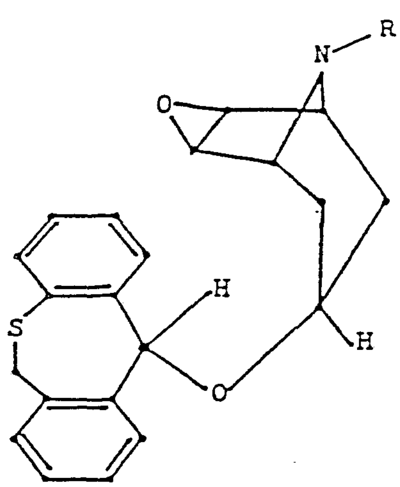

(Ia)

au moyen d'agents de méthylation usuels de formule générale IV

$$CH_3—X \quad (IV)$$

dans laquelle X a les significations indiquées plus haut.

6. Procédé pour la préparation de composés de formule générale Ia

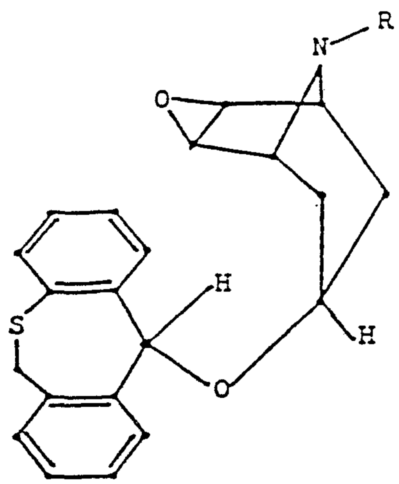

(Ia)

caractérisé en ce qu'on fait réagir des composés de formule générale II

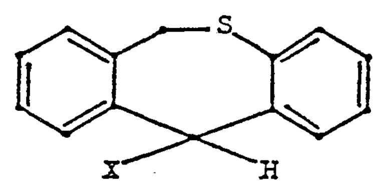

(II)

dans laquelle X représente un groupe facilement clivable, avec des composés de formule générale III

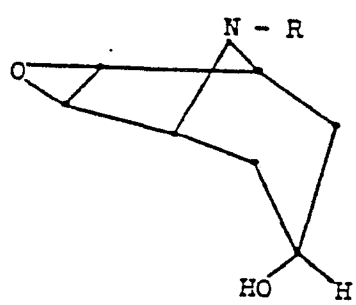

(III)

dans laquelle R a la signification indiquée plus haut.

7. Préparations pharmaceutiques, caractérisées par une teneur en composé(s) de formule générale I.

8. Procédé pour l'obtention de préparations pharmaceutiques selon la revendication 7, caractérisé en ce qu'on mélange de manière usuelle un ou plusieurs composés de formule générale I avec des adjuvants et/ou excipients galéniques usuels et transforme en médicaments.

9. Utilisation de composés de formule générale I pour la préparation de médicaments broncholytiques.